# EUROPEAN PATENT SPECIFICATION

(11) **EP 2 667 887 B1**
(45) Date of publication and mention of the grant of the patent: **16.12.2015**
(21) Application number: 12732889.6
(22) Date of filing: 26.01.2012
(51) Int. Cl.: A61K 38/06, A61P 1/04

(54) **PHARMACEUTICAL COMPOSITION COMPRISING THE PEPTIDE GLU-ASP-GLY AND USE FOR TREATING HELICOBACTER PYLORI INDUCED GASTRODUODENAL DISEASES**
PHARMAZEUTISCHE ZUSAMMENSETZUNG MIT DEM PEPTID GLU-ASP-GLY UND VERWENDUNG DAVON ZUR BEHANDLUNG VON DURCH HELICOBACTER PYLORI INDUZIERTEN MAGEN-DARM-ERKRANKUNGEN
COMPOSITION PHARMACEUTIQUE COMPRENANT LE PEPTIDE GLU-ASP-GLY ET UTILISATION POUR LE TRAITEMENT DE TROUBLES GASTRODUODÉNAUX INDUITS PAR HELICOBACTER PYLORI

(30) Priority: 27.01.2011 RU 2011103052; 12.12.2011 EA 201101622
(43) Date of publication of application: 04.12.2013
(73) Proprietor: «Garmonia», Ltd., St.Petersburg 197022 (RU)
(72) Inventor: KHAVINSON, Vladimir Khatskelevich, St.Petersburg, 197110 (RU); KVETNOI, Igor Moiseevich, St.Petersburg, 197183 (RU); POLYAKOVA, Victoria Olegovna, St.Petersburg, 197183 (RU); RYZHAK, Galina Anatolievna, St.Petersburg, 198205 (RU); KOZLOV, Lenar Vasilevich, St.Petersburg, 194044 (RU)
(74) Representative: Bohest AG
(86) International application number: PCT/RU2012/000035
(87) International publication number: WO 2012/102645

(56) References cited:
- WO-A2-2007/136295
- TUTEL'YAN V A ET AL: "Physiological role of short peptides in nutrition", BULLETIN OF EXPERIMENTAL BIOLOGY AND MEDICINE 20030101 US LNKD- DOI:10.1023/A:1023467622252, vol. 135, no. 1, 1 January 2003 (2003-01-01), pages 1-5, XP002682142, ISSN: 0007-4888

## Description

### Technical Field

The invention relates to medicinal means for treatment of *Helicobacter pylori-induced* gastroduodenal diseases. In particular, it relates to compounds intended for use in pharmaceutical industry and is directed to a means based on a pharmaceutical composition, which comprises a peptide having a specific activity as its active substance, which means may be used in medicine for treatment of *Helicobacter pylori-induced* gastroduodenal diseases.

The present disclosure uses the following terminology that is common in this field, as well as the following acronyms.

*Helicobacter pylori* is a small (2,5-0,4 x 0,5 µm) gram-negative bacterium that is an important pathogenic microorganism, which induces gastroduodenal diseases in humans. Colonization of gastric epithelial tissue by these bacteria causes development of inflammation and contributes to progressing chronic gastritis with a significantly high risk of a developing peptic gastric ulcer. Basing on an epidemiologic study of *Helicobacter pylori* the World Health Organization (WHO) has classified it as a Group 1 (definite) human carcinogen.

Gastroduodenal diseases associated with *Helicobacter pylori* infection are: gastritis, peptic ulcer, gastric and duodenal ulcer disease, gastric atrophy, intestinal metaplazia, non-ulcer dyspepsia, MALT lymphoma.

Mitochondrial mechanism of apoptosis is initiated mainly by different noci-influences causing increased permeability of the mitochondrial membrane and certain mitochondrial proteins emerging into the cytoplasm, in particular cytochrome C that binds with APAF-1 protein and stimulates formation of its oligomers. This, in turn, causes a recruitment of procaspase-9 molecules to the resulting complex, their aggregation, autoprocessing and generation of an active complex of caspase-9. At the next stage recruitment of procaspase-3 molecules to this complex occurs, as well as their processing into active forms that split key targets and cause apoptosis.

Apoptosis is a programmed or induced form of cell death, which is manifested in reduced cell size and chromatin condensation and fragmentation, condensation of outer and cytoplasmatic membranes without cell matter emerging into the environment. Apoptosis is a common biological mechanism responsible for supporting constancy of cell population quantities, as well as morphogenesis and discarding of deficient cells. Disordered apoptosis regulation leads to occurrence of different diseases related to intensification or, on the contrary, inhibition of apoptosis.

An effect of preventing induced cell death, leading to normalized apoptosis regulation is referred to as an anti-apoptotic effect.

### Background Art

It should be noted that the level of infection with *Helicobacter pylori* among the population of different countries varies from 50 to 90 %. Furthermore, the majority of subjects show asymptomatic carriage, while in 10% of cases helicobacter infection leads to the development of an ulcerous effect. It was experimentally shown that *Helicobacter pylori* is capable of enhancing apoptosis not only in endothelial cells but also in macrophages and T-lymphocytes, which leads to elimination of antigen-specific cells, reduces the efficiency of immune protection mechanisms and contributes to infection persistency [A.A. Ostanin et al., Characteristics of apoptosis and functional activity of lymphocytes in patients with ulcer disease. SO RAMN Bulletin, No.1 (111), 2004].

Antibiotic clarithromycin is widely used in the treatment of microbacterial infections to eradicate H. pylori genus bacteria [Cellini L., Marzio L. Rifabutin based triple therapy for eradication of H. pylori primary and secondary resistant to tinidazole and clarithromycin. - Dig Liver Dis. 2005Jan;37(1):33-8]. Klacid medication (clarithromycin, clarithrocine) is a synthesized antibiotic used for eradication of helicobacter infection [Vidal Reference Book. Medicinal Preparations in Russia. Reference Book. - M.: AstraPharmService, 2010 - pp. 657-660].

However, it should be noted that the use of Klacid medication has negative side effects, among them digestive system related ones: vomit, stomatitis, epigastric pains, glossitis, nausea, taste mutations, tongue discoloration, mycotic lesions of oral cavity mucous membrane, pseudomembranous colitis, diarrhea; nervous system related ones: vertigo, confused consciousness, headache, anxiety, anxiety dreams, insomnia, entotic sound, hallucinations, disorientation, depersonalization and psychosis, hearing loss; cardiovascular system related ones: elongated QT interval, ventricular fibrillation or flutter, tachycardia. Laboratory indications: hypoglycemia, transient increases in hepatic transaminase activity, thrombocytopenia and leucopenia. Allergic reactions: skin rash, nettle rash, in individual cases - Steven-Johnson syndrome and anaphylactic shock.

In the meantime, besides traditionally used medicinal means there are also known certain compounds, in particular peptide compounds, that are suitable for combating *Helicobacter pylori* bacteria.

There are known certain compounds (benzimidazole and imidazopyridin derivatives) suitable for combating *Helicobacter pylori* bacteria, which are disclosed in international applications WO 94/13290, WO 94/19346, WO 95/01351, WO 95/15324, WO 96/00224, WO 95/34554, WO 95/34553, WO 96/02534.

There is known a recombinant polypeptide that is a surface antigen to *Helicobacter pylori* having a molecular weight of approximately 29 kDa, as well as nucleic acid fragments coding said antigen [patent RU 2195463]. The recombinant polypeptide disclosed in RU 2195463 may be used for diagnosing *Helicobacter pylori* infections, as well as for producing vaccine compositions capable of triggering an immune protective response to said infection. Recombinant polypeptides are useful not only for diagnosing infections caused by *Helicobacter pylori,* but also for prophylactic application that would initiate (trigger) an immune protective response to such infections.

There is known a use of peptide with formula (I): ((X)l(Y)m)n, wherein the peptide comprises from 3 to 200 amino acids and where l, m and n are integers from 0 to 10; X and Y, which may be the same or different, are cationic amino acids selected from arginine and lysine, to produce medicinal means for treatment of a microbial infection [patent RU 2396273]. In the preferred aspect of the invention of RU 2396273, a use of the peptide for producing a medicinal means for treatment of a microbial infection is proposed. "Microbial infection" therein means an infection caused by a bacterial pathogen that may originate from bacterial genuses including Helicobacter spp., e.g. *Helicobacter pylori.*

There is known a use of (S)-(+)-2-[4-(2-fluorobenzyloxy)-benzylamino]propanamide as an anti-inflammatory agent, as well as a pharmaceutical composition having an anti-inflammatory activity, which includes a pharmaceutically acceptable additive, and (S)-(+)-2-[4-(2-fluorobenzyloxy)-benzylamino] propanamide as an active substance in an amount that is effective in reducing or preventing inflammation [RU 2396251]. Inflammatory states in mammals, in particular humans, which may be treated by administering one or more α-aminoamide compounds of said formula, include gastrointestinal diseases, in particular ulcers, gingivitis, Crohn's disease, atrophic gastritis, gastritis varialoforme, ulcerative colitis, celiac disease, localized ileitis, peptic ulcers, pyrosis and other gastrointestinal lesions, e.g., by *Helicobacter pylori.*

There is known a polypeptide that is homologous to a polypeptide expressed by Eurotinium amstelodami, which reveals an antimicrobial activity and may used as a treatment or prophylactic means in humans [patent RU 2393224]. Antimicrobial polypeptides disclosed in RU 2393224 are also suitable for use in compositions for *in vitro* elimination of microbes, in particular when it is undesirable to use a plurality of commonly used antibiotics. For example, antimicrobial polypeptides according to the invention may be added to animal and/or human food preparations; or they may be included as additives in cell cultures *in vitro* to prevent excessive growth of microbes in a tissue culture. In doing this, the sensitivity of a particular germ to elimination by means of antimicrobial peptides may be determined by means of an *in vitro* analysis. Germs of interest include Helicobacter sp., for example *Helicobacter pylori.*

There is known a use of allylamine derivatives such as terbinafine in producing a medicinal means for treatment of *Helicobacter pylori* infection or related diseases [patent RU 2193402].

There is known a use of quinolon- and naphtiridine carboxylic acid derivatives, which are substituted in the 7^{th} position with 2-oxa-5,8-diazabicyclo-[4,3,0]-non-8-yl radical, as well as salts thereof for treatment of *Helicobacter pylori* infections and related gastroduodenal diseases [patent EA 2477].

Among drawbacks of known means and compositions one may note their non-specific effect.

There is known a peptide glutamyl-aspartyl-glycine with general formula: H-Glu-Asp-Gly-OH [Register No. RN-75007-24-8 according to STN int. BD Registry Chemical abstract].

It is also known that peptide H-Glu-Asp-Gly-OH has a stress-protective effect in animals [patent RU 2304444, 2006, and corresponding WO 2007/136295].

Besides, it is known that peptide H-Glu-Asp-Gly-OH has an anti-tumor effect [patent RU 2362579, 2009].

Further experimental studies of peptide glutamyl-aspartyl-glycine with general formula: H-Glu-Asp-Gly-OH revealed its previously unknown property consisting in that it exerts a specific activity that is manifested in an inhibiting effect on mitochondrial mechanisms of *Helicobacter pylori-*induced apoptosis in gastric epitheliocytes.

Taking into account the high incidence of *Helicobacter pylori* infection-related gastroduodenal diseases (according to WHO assessments, approximately 50% of the Earth population are carriers of *Helicobacter pylori*), development of new groups of preparations, in particular biologically active compounds, which have a specific activity, is important.

### Disclosure of Invention

The present invention has set and achieved an objective of obtaining a pharmaceutical composition having a specific activity, for use thereof in the treatment of *Helicobacter pylori*-induced gastroduodenal diseases, by inhibiting mitochondrial mechanisms of *Helicobacter pylori*-induced apoptosis.

The technical result of the invention consists in the peptide glutamyl-aspartyl-glycine with formula: H-Glu-Asp-Gly-OH revealing a specific activity consisting in an inhibiting effect on mitochondrial mechanisms of apoptosis in gastric epitheliocytes, induced by *Helicobacter pylori,* in case of its use as an active substance in a pharmaceutical composition for use in the treatment of gastroduodenal diseases caused by *Helicobacter pylori,* which, when administered to a subject in need thereof, exerts a protective effect on mitochondrial mechanisms of *Helicobacter pylori*-induced apoptosis and prevents the germ from affecting the cells by means of inhibiting the process of *Helicobacter pylori*-induced apoptosis.

To achieve the above-stated objective and the specified technical result, there is proposed a group of inventions, which form one common inventive concept.

One of the aspects of the invention is directed to a pharmaceutical composition having a specific activity that is manifested in an inhibiting effect on mitochondrial mechanisms of *Helicobacter pylori-*induced apoptosis in gastric epitheliocytes, characterized in that it comprises an amount, effective on *Helicobacter pylori,* of peptide with formula H-Glu-Asp-Gly-OH as its active substance, and a pharmaceutically acceptable carrier.

According to the invention the pharmaceutical composition comprises the active substance in the amount of 1 µg/kg to 10 µg/kg.

According to the invention, the pharmaceutical composition exists in the form that is suitable for oral or parenteral administration.

The invention relates to the pharmaceutical composition for use in the treatment of *Helicobacter pylori*-induced gastroduodenal diseases.

Another aspect of the invention is directed to the peptide with formula H-Glu-Asp-Gly-OH as active substance for use in the treatment of *Helicobacter pylori*-induced gastroduodenal diseases.

Herein also described is a method of treatment for *Helicobacter pylori-induced* gastroduodenal diseases by inhibiting mitochondrial mechanisms of *Helicobacter pylori*-induced apoptosis in gastric epitheliocytes, comprising administering to a subject in need thereof an amount, effective on *Helicobacter pylori,* of peptide with formula H-Glu-Asp-Gly-OH in the dose of 1 µg to 10 µg per 1 kg of body weight orally, daily, 3 times a day for 10-20 days or parenterally daily, once a day for 10 days.

The administration is performed orally or parenterally.

The peptide glutamyl-aspartyl-glycine with formula: H-Glu-Asp-Gly-OH is obtained using a traditional method of peptide synthesis in a solution.

The possibility of objective achievement of the technical result using the invention is confirmed by reliable data including experimentally obtained information gathered using methods that are commonly used in the art.

Biological activity was studied in animals in an experimental model of gastric ulcer disease associated with *Helicobacter pylori.*

The term "pharmaceutical composition" includes various drug forms that comprise a peptide, which has a specific activity that is manifested in an effect on mitochondrial mechanisms of *Helicobacter pylori*-induced apoptosis, which may find therapeutic use as a means for prevention and treatment of a *Helicobacter pylori*-induced infection and gastroduodenal diseases related thereto.

The term "amount, effective on *Helicobacter pylori,* of the peptide" implies the use of such an amount of the active substance, which has been determined by *in vivo* experiment and which, in accordance with its quantitative characteristics of activity and toxicity, as well as in accordance with the knowledge of a person skilled in the art, should be effective in this drug form. Pharmaceutically acceptable carriers are selected from a group included in the State Pharmacopoeia, Edition XII, as additives used in production of a ready drug form that exists in the form of tablets or capsules (for oral administration) or in the form of a 0,9% sodium chloride solution (for parenteral administration).

To obtain the inventive pharmaceutical compositions, an effective amount of peptide H-Glu-Asp-Gly-OH as an active substance is mixed with a pharmaceutically acceptable carrier using compounding methods that are commonly used in pharmaceutics.

### Brief Description of the Drawings

The essence of the invention is illustrated by Figures.
Fig. 1 is a photograph of a fibroblast culture, where a shows 1 day of cultivation; b shows 3 days of cultivation; c shows 5 days of cultivation. Magnification x600.
Fig. 2 illustrates mitochondrial GPF expression in fibroblasts, where a shows the control values; b shows *Helicobacter pylori* effect; c shows the effect of peptide H-Glu-Asp-Gly-OH. Magnification x1000.
Fig. 3 illustrates mitochondrial GPF expression in fibroblasts, where a shows the control values; b shows *Helicobacter pylori* effect; c shows the effect of peptide H-Glu-Asp-Gly-OH. Magnification x1000.

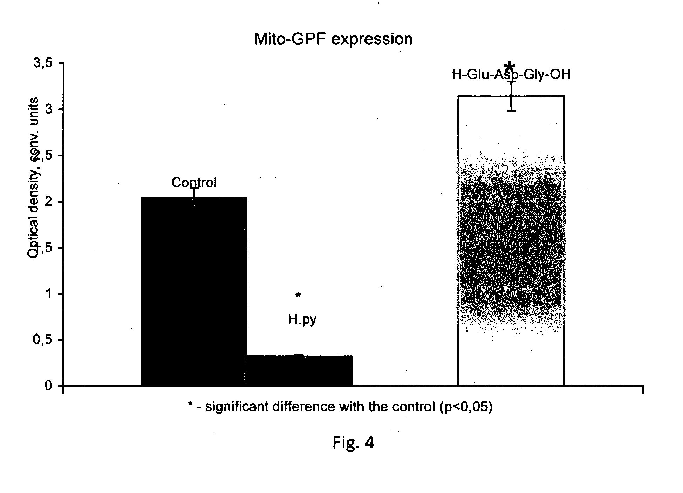

Fig. 4 shows the indices of mitochondrial GFP expression in fibroblasts under the effect of *Helicobacter pylori* and peptide H-Glu-Asp-Gly-OH.
Fig. 5 illustrates mitochondrial GPF expression in gastric epithelial cells. a shows the control values, b shows *Helicobacter pylori* effect, c shows the effect of peptide H-Glu-Asp-Gly-OH. Magnification x1000.
Fig. 6 shows the indices of optical density of mtxGFP expression in human gastric epitheliocyte cultures (H.Pyl-).
Fig. 7 shows the indices of optical density of mtxGFP expression in human gastric epitheliocyte cultures (H.Pyl+).
Fig. 8 shows the indices of mtxGFP expression area in human gastric epitheliocyte cultures (H.Pyl-).
Fig. 9 shows the indices of optical density of mtxGFP expression in human gastric epitheliocyte cells (H.Pyl+)
Fig. 10 illustrates proteins expression from an ulcer edge in tissue. *In vivo* study. Western blot. Expression level is in % with respect to control values taken as 100%.
Fig. 11 illustrates protein mRNA expression from a gastric ulcer edge in a mucous membrane. *In vivo* study. RT-PCR. Expression values are shown in conv. units reflecting the saturation of gel coloring.
Fig. 12 shows a pathomorphosis of an induced gastric ulcer. A shows the 7^{th} day (ulcer without administration of peptide H-Glu-Asp-Gly-OH); B shows the 21^{st} day (ulcer without administration of peptide H-Glu-Asp-Gly-OH); C shows the 21^{st} day (complete epithelization of the ulcer after the administration of peptide H-Glu-Asp-Gly-OH in the dose of 2,5 µg/kg).
Fig. 13 illustrates mitochondrial GFP expression in gastric epithelium on the 7^{th} day after ulcer induction.
Fig. 14 illustrates mitochondrial GFP expression in gastric epithelial cells on the 15^{th} day after ulcer induction. A shows intact animals; B shows ulcer induction; C shows the effect of peptide H-Glu-Asp-Gly-OH. Magnification x1000.
Fig. 15 illustrates mitochondrial GFP expression in isolated mitochondria of gastric epithelial cells on the 21^{st} day after ulcer induction. A shows intact animals; B shows ulcer induction; C shows the effect of peptide H-Glu-Asp-Gly-OH. Magnification x1200.
Fig. 16 illustrates mitochondrial GFP expression in gastric epithelium on the 15^{th} day after ulcer induction.
Fig. 17 illustrates mitochondrial GFP expression in gastric epithelium on the 21^{st} day after ulcer induction.

The invention is also illustrated by the Examples of: synthesis of peptide glutamyl-aspartyl-glycine with general formula: H-Glu-Asp-Gly-OH **(Example 1),** study of the effect of pharmaceutical composition comprising the peptide H-Glu-Asp-Gly-OH on molecular and cellular mechanisms of apoptosis **(Example 2),** study of biological activity and effects of peptide H-Glu-Asp-Gly-OH on mitochondrial mechanisms of apoptosis in gastric epitheliocytes **(Example 3),** *in vivo* study of biological activity and effects of peptide H-Glu-Asp-Gly-OH on mitochondrial mechanisms of apoptosis in gastric epitheliocytes induced by *Helicobacter pylori* germ **(Example 4),** study of toxicity of peptide H-Glu-Asp-Gly-OH **(Example 5).**

### Example 1. Synthesis of peptide H-Glu-Asp-Gly-OH

1. Compound name: **glutamyl-aspartyl-glycine.**
2. Structural formula: **H-Glu-Asp-Gly-OH** 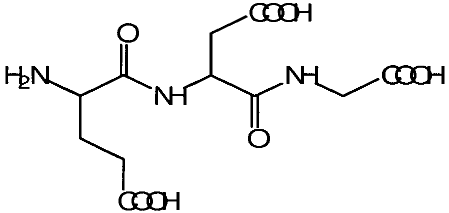
3. General formula without ion pair: C₁₁H₁₇N₃O₈.
4. Molecular mass without ion pair: 319,27.
5. Ion pair: acetate.
6. Appearance: white amorphous powder without smell.
7. Method of synthesis: the peptide was obtained using a traditional method of synthesis in a solution according to the following scheme: 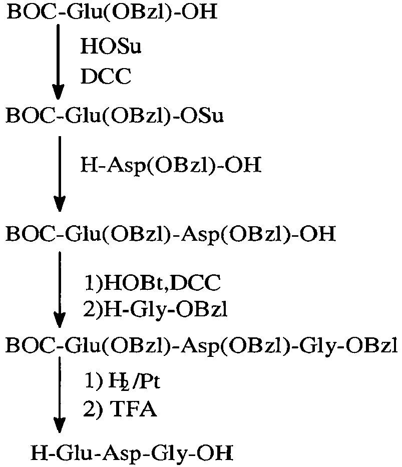
   BOC - tert.butyloxycarbonyl group,
   OSu - N-oxysuccinimide ester,
   DCC - N,N'-dicyclohexylcarbodiimide,
   OBzl - benzyl ester,
   TFA - trifluoracetic acid,
   HOBt - N-oxybenzotriazol.

Characteristics of the ready substance:
- Base substance content: 97,93% (by HPLC, 220 nm),
- TLC - individual, R_{f}=0,45 (acetonitrile-water 1:2),
- Humidity content: 6%,
- pH of 0,01% solution: 4,9,
- Specific rotary power: [α]_{D}²²: -31° (c=1, H₂O), "Polamat A", Carl Zeiss Jena.

### Example of synthesis:

### 1) BOC-Glu(OBzl)-OSu, N-oxysuccinimide ester of N-tert.butyloxycarbonyl-(γ-benzyl)glutamic acid (I).

N-tert.butyloxycarbonyl-(γ-benzyl)glutamic acid BOC-Glu(OBzl)-OH (33,7 g, 0,1 mole) was dissolved in 50 ml of N,N'-dimethylformamide, cooled to -10°C; cooled (4-6°C) solutions of N,N'-dicyclohexylcarbodiimide (23,0 g, 0,11 mole) were added while stirring in 30 ml of N,N'-dimethylformamide and N-hydroxysuccinimide (13,0 g, 0,11 mole) in 20 ml N,N'-dimethylformamide. Reactive mixture was stirred for 12 hours, cooled with ice, and then for 24 hours at room temperature. The residue N,N'-dicyclohexylurea was filtered out and the obtained solution of activated ester was used without extracting during the next step.

### 2) BOC-Glu(OBzl)-Asp(OBzl)-OH, N-tert.butyloxycarbonyl-(γ-benzyl)glutamyl-(β-benzyl)aspartate (II).

(β-benzyl) asparaginic acid H-Asp(OBzl)-OH (28,0 g, 0,12 mole) and 36 ml (0,12 mole) of triethylamine were suspended in 50 ml of N,N'-dimethylformamide and stirred for 1 hour. Then the solution of activated ester **BOC-Glu(OBzl)-OSu (I)** obtained at the previous step was added in portions. The reactive mixture was stirred at room temperature for 48 hours. Then the mixture was acidated by 0,5 N sulphur acid up to pH 2-3 and extracted with ethyl acetate 4x50 ml. The extracts were combined and washed sequentially with 0,5 N H₂SO₄ 3x50 ml, water 2x50 ml, 5% NaHCO₃ solution 2x50 ml, water 2x50 ml, saturated NaCl solution 2x50 ml. Organic layer was dried out over Na₂SO₄, the solvent was evaporated in vacuum, and the residue was crystallized under hexane. 50 g of product was obtained (92%). R_{f} = 0,34 (benzene-acetone 2:1).

### 3) BOC-Glu(OBzl)-Asp(OBzl)-Gly-OBzl (III), benzene ester of N-tert.butyloxycarbonyl-(γ-benzyl)glutamyl-(β-benzyl)aspartyl-glycine (III).

2,7 g (5 mmole) of the dipeptide and 0,95 g (7 mmole) of oxybenzotriazole was dissolved in tetrahydrofuran (10 ml) and cooled to the temperature of -10 °C. 1,44 g (7 mmole) of dicyclohexylcarbodiimide were dissolved in 5 ml of tetrahydrofuran and cooled to the same temperature. 3,4 g (10 mmole) of benzene ester tosilate of glycine were dissolved in 10 ml of tetrahydrofuran, 1,4 ml (10 mmole) of triethylamine were added and cooled to the same temperature. While cooling in an ice bath and stirring intensely, the solutions of the dipeptide with oxybenzotriazol and of the carbodiimide were combined, in 10 minutes a glycine benzyl ester tosilate solution was added. The reactive mixture was stirred for 3 hours while cooling with ice and for 24 hours at room temperature. The fallout dicyclohexylurea was filtered out, filtrate was evaporated in vacuum, the residue was dissolved in ethyl acetate (100 ml). The solution was washed sequentially in 0,5 N sulfur acid, water, 5% sodium bicarbonate solution, water, dried over waterless sodium sulfate. Ethyl acetate was evaporated in vacuum, the residue was crystallized in a ethyl acetate / hexane system. Recrystallization from isopropanol was performed. After drying in vacuum, 2,74 g of the product was obtained (85%). R_{f} = 0,78 (benzene-acetone 1:1).

### 4) H-Glu-Asp-Gly-OH (IV), glutamyl-aspartyl-glycine.

Protected tripeptide BOC-Glu(OBzl)-Asp(OBzl)-Gly-OBzl (III) (2,7 g) was dissolved in a methyl spirit - water mixture (4:1) (50 ml) and hydrated over catalyst Pd/C (5%) for 4 hours. The catalyst was filtered out, solvent was evaporated in vacuum, residue was dried in vacuum over KOH and P₂O₅. Then the product was dissolved in 15 ml of chlorous methylene - trifluoracetic acid mixture (5:1) and kept at room temperature for 2 hours. The completeness of unblocking reaction was monitored by means of TLC in an acetonitrile-water system (1:3). The solvent was evaporated in vacuum, the residue was dried in vacuum over KOH.

For purifying, 300 mg of the preparation were dissolved in 4 ml of 0,01% trifluoracetic acid and subjected to high performance liquid chromatography on a reverse phase column 50x250 mm Diasorb-130-C16T, 7 µm. Chromatographic apparatus Beckman System Gold, 126 Solvent Module, 168 Diode Array Detector Module. Chromatography conditions: A: 0,1 % TFA; B: MeCN/0,1% TFA, gradient B 0 → 50% in 100 minutes. Sample volume 5 ml, detection at 215 nm, scanning 190-600 nm, flow rate 10 ml/minute. Basic peak fraction was selected. The solvent was evaporated in vacuum at the temperature not higher than 40°C, evaporation was repeated several times (5 times) with 10 ml of 10% acetic acid solution.

Eventually the residue was dissolved in 20 ml of deionized water and lyophilized. 150 mg of purified preparation in the form of white amorphous powder without smell was obtained.

### 5) Analysis of the ready substance

- Main substance content was identified by means of HPLC on a Phenomenex C 18 LUNA column 4,6x150 mm. A: 0,1% TFA, B: MeCN; grad.B 0-100% in 10 min. Flow rate 1 ml/minute. Detection at 220 nm, scanning at 190-600 nm, sample volume 20 µl. Main substance content 97,93 %.
- TLC: individual, R_{f} = 0,45 (acetonitrile-water 1:2, Sorbfil plates, silicagel 8-12 µm, developing by chlorine/benzidine).
- Moisture content: 6% (gravimetrically, by weight loss in drying 20 mg at 100°C).
- pH of 0,01 % solution: 4,9 (potentiometrically).
- Specific rotary power: [α]_{D}²²: -31° (c=1, H₂O), "Polamat A", Carl Zeiss Jena.

Main substance (or active substance) produced in the form of a dry preparation, may be converted into the pharmaceutical composition according to the invention by adding a pharmaceutically acceptable carrier.

To obtain a ready drug form of a means for preventing or treatment of a *Helicobacter pylori-*induced infection pharmaceutically acceptable carriers are selected from a group of those included in the State Pharmacopoeia, Edition XII, for additives used for producing a ready drug form of tablets or capsules (for oral administration) or 0,9% sodium chloride solution (for parenteral administration).

### Example 2. Study of the effect of pharmaceutical composition comprising the peptide H-Glu-Asp-Gly-OH on Molecular and cellular mechanisms of apoptosis

A study of the effect of a pharmaceutical composition comprising the peptide H-Glu-Asp-Gly-OH as an active substance on molecular and cellular mechanisms of apoptosis was carried out on a wild type of mice embryonic fibroblasts (MEF+/+, knockout mice line LMNA). Cells were cultivated at 37°C in a damp incubation medium containing 5% CO2 in DMEM (Dulbecco's Modified Eagle's Medium, ICN Biomedicals) and 10% calf embryonic serum. Preliminary sorting of cells for passaging in a 1:3-1:5 ratio was performed using a mixture of 0.125% trypsin, 0.02M EDTA (ethylenediaminetetraacetic acid), 0.02% glucose dissolved in a phosphate buffer. Cells were passaged in a culturing medium for 5 days and then taken for the study.

On the 5^{th} day after the beginning of passaging of the cells peptide H-Glu-Asp-Gly-OH was added in the culturing medium in the dose of 10 µM, and in 30 minutes the cells were taken for study.

*Helicobacter pylori* (Curtin Matheson Scientific Inc, strain Cag J117, 100 microbial cells) was added in the culturing medium, in which the mouse embryonic fibroblasts were cultivated, on the 5^{th} day of cultivation, then incubated for 7 hours for destruction of mitochondria (a known effect of this germ causes rupture of mitochondrial membrane, accompanied by a formation of reactive oxygen forms and a release of apoptogenic factors that are a signal for activation of a final effector stage of apoptosis), after which the fibroblasts were taken for study.

On the 5^{th} day of cultivation of mouse embryonic fibroblasts peptide H-Glu-Asp-Gly-OH was also administered in the culturing medium in the dose of 10 µM 30 minutes before the introduction of *Helicobacter pylori* in the culturing medium, and after incubation for 7 hours together with *Helicobacter pylori* the fibroblasts were taken for study.

The status of isolated mitochondria was evaluated using immunofluorescent confocal laser microscopy.

Cell lysis to isolate mitochondria was performed in an isotonic buffer (200 mM mannitol, 70 mM sucrose, 1 mM EGTA - ethylene glycol tetraacetic acid, 10 mM HEPES - 4-(2-hydroxyethyl)-1-piperazineethanesulfonic acid), pH 6.9) for 30 minutes. Non-disrupted cells, nuclei and membranes were removed by centrifuging (1000 g, 5 minutes). To obtain an enriched fraction of mitochondria, the supernatant fluid was additionally centrifuged (12000 g, 20 minutes). The fraction containing isolated mitochondria was placed in two-layer glass slides with 4.3 mm² chambers (Nalge Nunc Inc.). In each chamber, 2.0 µl of monoclonal antibodies to green mitochondrial fluorescent protein (mito-GFP, Clontech Laboratories, Inc.) were added and incubation for 1 hour was carried out.

Confocal microscopy of mitochondria with optical density evaluation was carried out in an inverted confocal microscope Olympus Fluoview CM FV300-IX70 using an apochromatic lens 606 UPlan (Olympus). A 488 nm excitation wave of argon laser was used in each chamber to specify mito-GFP fluorescence that verifies the structure of mitochondria.

Observation of the fibroblast culture in the control has shown that the monolayer was integral and equally distributed in 24 hours, the fibroblasts retained normal shape and size. Monolayer density was 774 cells per mm². Culture duplication time was 24 hours. Monolayer type and cell structure were not different from the normal ones during all 5 days of observation. Monolayer density on the 3^{rd} day made 1519 cells per mm².

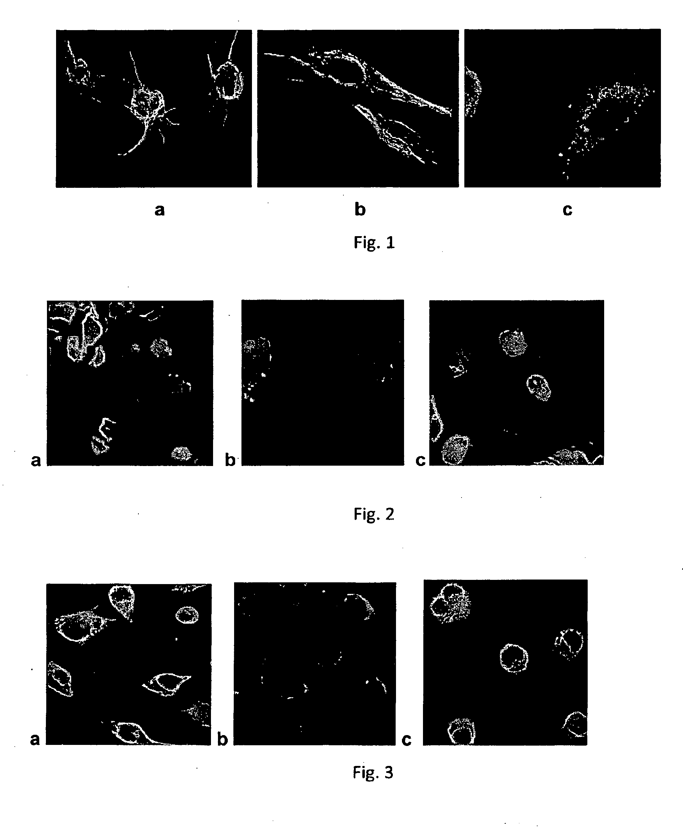

On the 5^{th} day of experiment the cells reached the saturation density that is optimal for this culture, which made 2020 cells per mm², which brought about the culture's transition to a stationary growth phase, in which the fibroblasts had an elongated shape that is typical for these cells, 2-4 arms, cellular and nuclear membranes were distinctly contoured (Fig. 1), cell cytoplasm was homogenous.

Most cells had 1 centrally located nucleus of a regular round shape with 1 or 2 nucleoli. In this phase the cells were in an optimal state for a study of their structure and functions.

*Helicobacter pylori* induced the fragmentation of mitochondria, which disintegrated into fragments of different shapes and sizes, mitochondrial membrane and cristae were disrupted, fluorescence intensity in mitochondrial GFP was reduced, which affected the indices of optical density (Fig .2-4).

Under an effect of the pharmaceutical composition containing H-Glu-Asp-Gly-OH peptide integrity of outline and structure of mitochondria was observed, and luminescence of mitochondrial GFP even exceeded the control indices in terms of optical density values, which points out the presence of pronounced capabilities of prevention of *Helicobacter pylori-induced* mitochondrial apoptosis disorder in the studied peptide.

Thus, the studies made it possible to specify the mechanism of biological activity of the pharmaceutical composition comprising H-Glu-Asp-Gly-OH peptide and of its effect on the mitochondrial way of initiating apoptosis.

A pronounced anti-apoptotic effect of the pharmaceutical composition comprising the peptide H-Glu-Asp-Gly-OH that was identified in the course of studies, said effect being implemented by means of blocking the mitochondrial cascade of pro-apoptosis caspases production, shows a high potential of said composition for its applied use in the therapy of pathologies related to cell death or distortion of normal way of cell differentiation, especially for the treatment of ulcerous disease of the stomach and duodenum (as well of a possible tumoral metaplasia that is related to this pathology) associated with *Helicobacter pylori,* as the peptide hinders the germ's impact on the cells by inhibiting mitochondrial mechanisms of *Helicobacter pylori*-induced apoptosis in gastric epitheliocytes, thus revealing a strong anti-apoptotic effect on cells.

### Example 3. A study of biological activity and effects of peptide H-Glu-Asp-Gly-OH on Helicobacter pylori-induced mitochondrial mechanisms of apoptosis in gastric epitheliocytes

To evaluate the biological activity and effects of peptide H-Glu-Asp-Gly-OH on mitochondrial mechanisms of apoptosis in gastric epitheliocytes, induced by *Helicobacter pylori* germs, human gastric epithelium cells SGC7901 were studied. The cells were cultivated at 37°C in a damp incubation medium containing 50 mL/L CO₂ in DMEM (Dulbecco's Modified Eagle's Medium, ICN Biomedicals) and 10% calf embryonic serum. A preliminary separation of cells for passaging was carried out in the ratio of 1:3-1:5 using a mixture of 0.125% trypsin, 0.02M EDTA (ethylenediaminetetraacetic acid), 0.02% glucose dissolved in a phosphate buffer. The cells were placed on a 24-well plate in the amount of 10⁶ cells per well. The cells were passaged in a cultivation medium for 5 days and then taken for study.

On the 5^{th} day after the beginning of cell passaging the peptide H-Glu-Asp-Gly-OH in the dose of 10 µM was administered in the cultivation medium, and in 30 minutes of incubation the cells were taken for study. Klacid antibiotic (clarithromycin, Abbott) in the dose of 500 ng/mL was administered to the cultures in the same conditions as positive control.

*Helicobacter pylori* (Curtin Matheson Scientific Inc, strain Cag J117, 100 microbial cells) was added in the cultivation medium, in which human gastric epitheliocytes were cultivated, on the 5^{th} day of cultivation, incubated for 7 hours for destruction of mitochondria (a known effect of this germ causes rupture of mitochondrial membrane, accompanied by a formation of reactive oxygen forms and a release of apoptogenic factors that are a signal for activation of a final effector stage of apoptosis), after which the epitheliocytes were taken for study.

On the 5^{th} day of human gastric epitheliocytes' cultivation peptide H-Glu-Asp-Gly-OH was administered in the cultivation medium in the dose of 10 µM 30 minutes before the administration of *Helicobacter pylori* in the cultivation medium, in 7 hours of cell incubation after the addition of *Helicobacter pylori* (strain Cag J117, 100 microbial cells) in the medium the epitheliocytes were taken for study. Klacid antibiotic (clarithromycin, Abbott) in the dose of 500 ng/mL was administered to the cultures in the same conditions as positive control.

The status of isolated mitochondria was evaluated using immunofluorescent confocal laser microscopy.

Cell lysis for isolation of mitochondria was carried out in an isotonic buffer (200 mM mannitol, 70 mM sucrose, 1 mM EGTA - ethylene glycol tetraacetic acid, 10 mM HEPES - *4*-*(2*-hydroxyethyl)-1-piperazineethanesulfonic acid), pH 6.9) for 30 minutes. Non-disrupted cells, nuclei and membranes were separated by centrifuging (1000 g, 5 minutes). To obtain an enriched fraction of mitochondria, the supernatant fluid was additionally centrifuged (12000 g, 20 minutes). The fraction containing isolated mitochondria was placed in two-layer glass slides with 4.3 mm² chambers (Nalge Nunc Inc.). 2.0 µl of monoclonal antibodies to green mitochondrial fluorescent protein (mito-GFP, Clontech Laboratories, Inc.) were added in each chamber and incubated for 1 hour.

Confocal microscopy of mitochondria with optical density evaluation was performed in an inverted confocal microscope Olympus Fluoview CM FV300-IX70 using an apochromatic lens 606 UPlan (Olympus). A 488 nm excitation wave of argon laser was used in each chamber to specify mito-GFP fluorescence that verifies the structure of mitochondria.

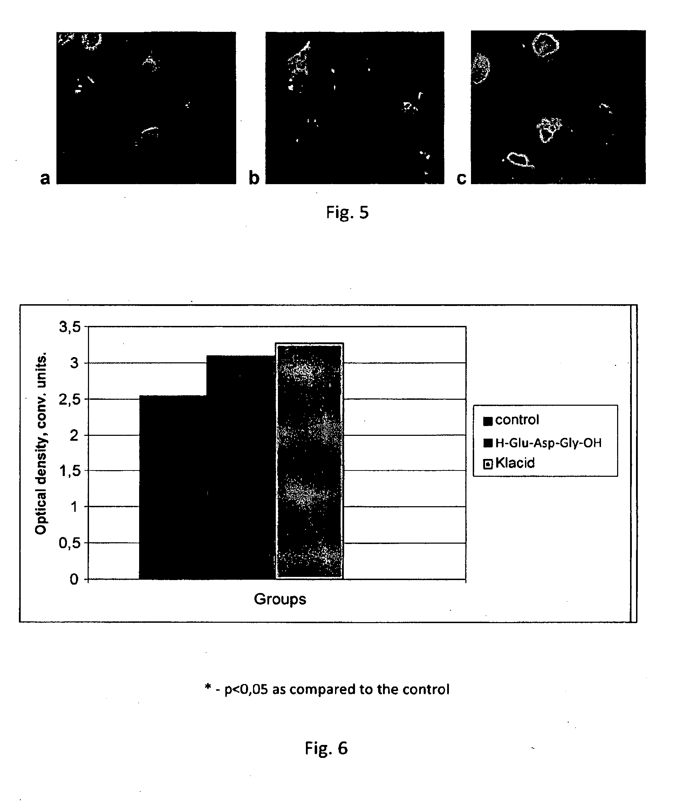

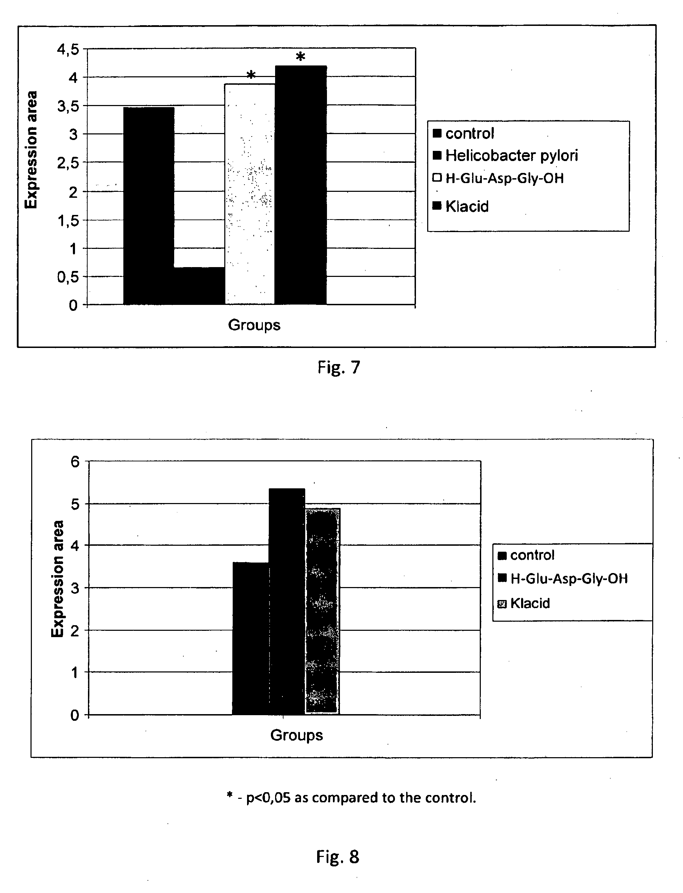

In the control, mitochondria in gastric epithelial cells had a normal round or oval shape with distinct fluorescence of cristae and matrix. Expression of mitochondrial GFP was pronounced and made 2.57±0.23 conv. units by optical density value and 3.42±0.19% by mean expression area value (Fig. 5-9). *Helicobacter pylori* administration in the cultivation medium caused a distinct fragmentation of mitochondria accompanied by disintegration of mitochondrial membrane and cristae and reduced intensity of mitochondrial GFP fluorescence, which caused an abrupt decline of marker expression indices (0.35±0.05 conv. units by optical density; 0.63±0.03% by mean expression area) (Fig.5-9). Under the effect of the pharmaceutical composition comprising the H-Glu-Asp-Gly-OH peptide, integrity of outline and structure of mitochondria was observed, and mitochondrial GFP expression was largely not changed as compared to the control values (2.48±0.17 conv. units by optical density; 3.84±0.20% by expression area), which points out the presence of pronounced capabilities of the studied peptide for prevention of *Helicobacter pylori*-induced mitochondrial apoptosis disorder when used in the pharmaceutical composition comprising the H-Glu-Asp-Gly-OH peptide. It should be also noted that the specified biological activity of the pharmaceutical composition comprising the peptide H-Glu-Asp-Gly-OH is comparable in terms of efficiency with the activity of the known antibiotic Klacid, which is used as antibacterial drug in the treatment of gastric pathologies associated with *Helicobacter pylori* germ.

### Example 4. in vivo study of biological activity and effects of peptide H-Glu-Asp-Gly-OH on mitochondrial mechanisms of apoptosis in gastric epitheliocytes induced by Helicobacter pylori germ

The study was carried out on 90 male Sprague Dawley rats (180-220 g) randomly subdivided into 9 groups (10 animals per group):
- Group 1 - intact rats (saline solution)
- Group 2 - modeled ulcer + saline solution
- Group 3 (6 subgroups) - modeled ulcer + H-Glu-Asp-Gly-OH
- Group 4 - modeled ulcer + Klacid antibiotic

Ulcer was modeled by three-time administration (in 4 hours) in the rat stomach of cysteamine-HCL (Aldrich, Milwannee, WI) by means of a probe in the dose of 25 mg per 100 g of body weight. The ulcer, approximately 28 mm² in size, appeared at the boundary between antral and fundal segments of the stomach 12 hours after the last administration. Simultaneously with the first administration of cysteamine-HCL a *Helicobacter pylori* culture (Curtin Matheson Scientific Inc, strain Cag J117, 100 microbial cells) was administered in the rat stomach through the same probe.

A means on the basis of the pharmaceutical composition comprising the studied peptide H-Glu-Asp-Gly-OH was administered subcutaneously in the dose of 1 µg per kg of animal body weight (group 3a), 2,5 µg per kg of body weight (group 3b) and 10 µg per kg of body weight (group 3c) in 0,5 ml of saline solution daily, for 5 days from the occurrence of the stomach ulcer.

The means on the basis of the pharmaceutical composition comprising the studied peptide H-Glu-Asp-Gly-OH was administered intragastrically (through a probe) in the dose of 1 µg per kg of animal body weight (group 3d), 2,5 µg per kg of body weight (group 3e) and 10 µg per kg of body weight (group 3f) in 0,5 ml of saline solution daily, for 5 days from the occurrence of the stomach ulcer.

Antibiotic Klacid (clarithromycin, Abbott) was administered intramuscularly in the dose of 10 mg in 1 ml of saline solution daily for 5 days from the occurrence of the stomach ulcer.

Material for study was taken from ulcer edge on the 7^{th} day (for western blot assay and polymerase chain reaction in real time - RT-PCR), as well as on the 7^{th}, 15^{th} and 21^{st} day for morphology study and confocal microscopy.

Molecular biology study was performed using the following molecules as signal molecules, the expression of which reflects biochemical disorders in the stomach walls and reparative changes in the process of development of ulcerous destruction: constitutive and inducible NO synthase (cNO and iNO respectively), main representative of heat shock protein family - HSP70, and NF-kappa b-p65 factor. Expression of these factors was studied using a western blot method according to a standard protocol with the following basic steps.

Samples were subjected to electrophoresis in 1-dimensional gel plates on nitrocellulose sheets with pore diameter of 0,45 µM (Millipore) using abrasive sponges (Scotch-Brite).

The plates were inserted in an electrophoresis chamber with a nitrocellulose sheet before the cathode. The chamber contained 0,7% acetic acid, voltage gradient 6 V/cm was supported for 1 hour. Sheets with electrophoresis stains were processed with 3% bovine serum albumin in saline solution (0,9% NaCl/10 MM tris-HCl, pH 7,4) for 1 hour at 40°C, then washed in saline solution and incubated with the desired antibodies (Biorad). Then the sheets were washed in saline solution (5 times for 30 minutes) and incubated with an indicator (second antibodies to immunoglobulins of primary antibodies).

Visualization of marked stains was performed by means of incubation with fluorescein isothiocyanate or goat anti-IgG bound with horseradish peroxidase (Fluca, 1:100) for 30 minutes at room temperature. When using fluorescein, the stains were photographed with a Polaroid camera in long-wave UV radiation using a yellow filter. When using peroxidase, wet stains were colored in a 25% guadianizidine (pH 7,4) solution for 20 minutes and then dried by means of a filter paper and photographed.

As the expression of said signal molecules is directly related to the level of synthesis of certain factors, RT-PCR method was used to evaluate mRNA expression of the following substances: superoxide dismutase, TNF-alpha and Cox-2.

RT-PCR was carried out in 25 µl of reactive mixture containing a buffer (40 MM tris-HCl pH 8,0, 2,5 MM MgCl2, 25 MM KCl); 20 pmole DNA, 1 act. unit of Taq-DNA polymerase; 0,5 pmole of each of the primers, each stained with own fluorochrome (donor and acceptor), and a respective amount of distilled water. RT-PCR was carried out in a DNA amplifier iCycler iQ (Bio-Rad) in the following conditions: denaturation of the double-stranded DNA in the first cycle was carried out at 94 °C for 30 seconds, then the primers were annealed and elongated at 45 °C for 10 seconds with fluorescence registration at the end of this stage; denaturation of the target product at 80 °C for 10 seconds, number of cycles - 25. Primer annealing temperature, as well as melting temperature of target products varied to a certain extent in different experiments depending on GC composition of the oligonucleotides in use.

Electrophoresis control of RT-PCR products was carried out in 8% polyacrylamide gel in a tris-acetate buffer at pH 7.8 in non-denaturation conditions, at voltage gradient of 4 V per 1 cm of gel length in a vertical-type apparatus for 4 hours.

Upon completion of electrophoresis the gel was stained with ethidium bromide and photographed in a photodocumentation system Gel Camera System (UVP, Inc., USA).

For a morphology study, pieces of gastric mucous membrane from the ulcer edge were fixed in 10% neutral formalin (pH 7.2), then the material was dried out in a Leica TP1020 automatic station (Leica) and covered with paraffin. 5 µm thick paraffin slices were placed on object plates covered with a poly-L-lysine film (Sigma). For a visual study the slices were stained with hematoxylin and eosin, as well as with picrofuxin by the van Gison method.

The status of isolated mitochondria was assessed using immunofluorescent confocal laser microscopy.

Cell lysis for the isolation of mitochondria was performed in an isotonic buffer (200 mM mannitol, 70 mM sucrose, 1 mM EGTA - ethylene glycol tetraacetic acid, 10 mM HEPES - 4-(2-hydroxyethyl)-1-piperazineethanesulfonic acid), pH 6.9) for 30 minutes. Non-disrupted cells, nuclei and membranes were separated by centrifuging (1000 g, 5 minutes). To obtain an enriched fraction, the supernatant fluid was additionally centrifuged (12000 g, 20 minutes). The fraction containing isolated mitochondria was placed in two-layer object plates with 4.3 mm² chambers (Nalge Nunc Inc.). 2.0 µl of monoclonal antibodies to green mitochondrial fluorescent protein (mito-GFP, Clontech Laboratories, Inc.) were added in each chamber, and incubation was carried out for 1 hour.

Confocal microscopy of mitochondria with optical density evaluation was carried out in an inverted confocal microscope Olympus Fluoview CM FV300-IX70 using an apochromatic lens 606 UPlan (Olympus). A 488 nm excitation wave of argon laser was used in each chamber to specify mito-GFP fluorescence that verifies the structure of mitochondria.

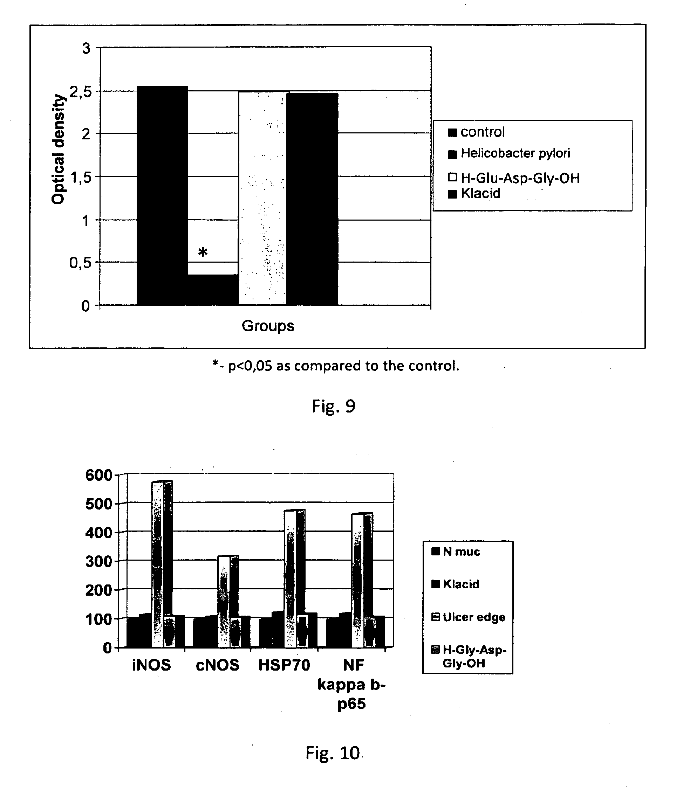

The use of western blot method showed that the expression of all studied signal molecules in mucous membrane samples from the edge of the gastric ulcer on the 7^{th} day after ulcer induction significantly increases (by 3-5 times) as compared to a normal mucous membrane taken from intact animals (Fig. 10). In this case the growth of expression of iNOS and NF kappa b-p65 factor is especially drastic.

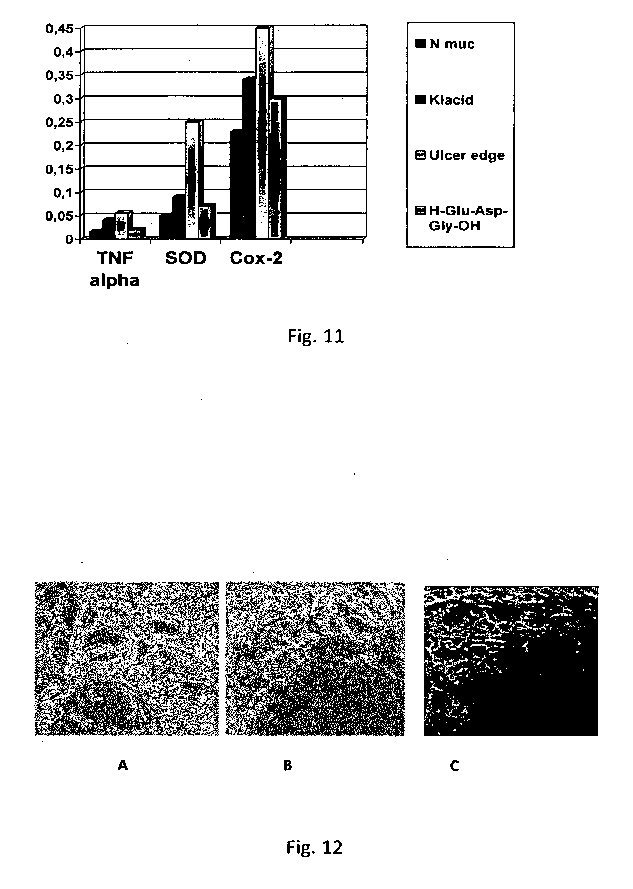

RT-PCR has shown that in case of ulcer induction the expression of mRNA that codes the synthesis of superoxide dismutase (SOD), TNF-alpha and Cox-2 factor increases by the 7^{th} day in mucous membrane samples from the edge of the ulcer (Fig. 11).

The study of biological activity of the means on the basis of the pharmaceutical composition comprising the peptide H-Glu-Asp-Gly-OH in the doses of 1 µg to 10 µg per kg of body weight has shown that it has a protective effect on the gastric mucous membrane, reducing an increased expression of both NO synthase forms and of factors HSP70 and NF kappa b-p65. The level of production of said signal molecules in the mucous membrane of the edge of the ulcer by the 7^{th} day from its induction is comparable with commonly normal indices.

The study of macroscopic and histological preparations has shown the following pathomorphosis pattern of the induced ulcer (Fig. 12).

In Group 2 (modeled ulcer + saline solution) the ulcers retained the same size on the 7^{th} day of the observation as on the 1^{st} day from the time of ulcer induction.

A perifocal edema was highly developed and ulcer walls were covered with fibrinoid necrosis patch with signs of a chronic inflammation and small erosions. A broad leukocytal necrosis layer was found on the ulcer floor, under which there was granulation tissue with an increased amount of thin-walled blood vessels and predominant fibroblasts.

In the deeper parts of granulation tissue collagen fibers with a partly regular pattern and a small amount of vessels were seen. In the layer underlying the necrosis zone and in deeper layers of the granulation tissue a pronounced diffuse inflammatory infiltration was observed with predominant neutrophils.

In Group 3 (administration of the means on the basis of the pharmaceutical composition comprising the peptide H-Glu-Asp-Gly-OH) by the 7^{th} day after ulcer induction a decrease both in the area of the ulcerous lesion and in its depth was observed, and the ulcer floor was cleaned up of necrotic masses, and inflammation around the ulcer was reduced.

Histology study of the gastric ulcer showed that the leukocytal necrosis layer has become thinner. The underlying layer consisted of a growing up granulation tissue with a large amount of vessels and moderate diffuse leukocytal infiltration, predominantly of histiocytes, lymphocytes and neutrophils. Cystically dilated glands with epitheliocyte proliferation were seen about the edges of the ulcerous lesion.

Such effect of the means on the basis of the pharmaceutical composition comprising the peptide H-Glu-Asp-Gly-OH was observed in all 6 subgroups of animal Group 3 - both in subcutaneous and in intragastric administration in 3 concentrations.

Morphological changes in Group 4 (administration of Klacid) were largely similar to the changes in Group 3.

On the 15^{th} day in Group 2 animals (ulcer + saline solution) the area of the ulcerous lesion was somewhat reduced, but the ulcer floor remained partly covered with a necrosis patch, although the signs of chronic inflammation became less notable.

After administration of the means on the basis of the pharmaceutical composition comprising the peptide H-Glu-Asp-Gly-OH (Group 3), subcutaneously (Groups 3a, 3b, 3c) or intragastrically (Groups 3d, 3e, 3f) in three different doses a significant decrease in ulcerous lesion area and depth was observed. Under the effect of the means on the basis of the pharmaceutical composition comprising the peptide H-Glu-Asp-Gly-OH partial epithelization of the edges of the ulcerous lesion could be confirmed already by the 15^{th} day. A thin leukocytal necrosis layer was observed in the ulcer floor.

As compared to the above-discussed observation time, granulation tissue growth and increased number of blood vessels were observed; collagen fiber pattern in the granulation tissue was more regular, inflammatory infiltration was less pronounced on the account of predominantly lymphoid and histiocyte type cells.

Leukocytal infiltration became fine-focal, hemostasis was reduced and the amount of newly formed microvessels was increased. Population of mast cells and macrophages increased. Mucous membrane assumed a uniformly pink color. The same changes were observed in Group 4 (Klacid).

By the 21^{st} day ulcerous lesions were still present in Group 2 animals. Ulcer floor was covered with a thin necrosis layer and diffusely infiltrated by leukocytes. In the underlying layer the growing granulation tissue showed a partly regular pattern of collagen fibers and a moderate inflammatory infiltration by histiocytes, lymphocytes and neutrophils. Cystically dilated glands covered with proliferating epithelium were observed in the ulcer edges.

In Group 3 (administration of the means on the basis of the pharmaceutical composition comprising the peptide H-Glu-Asp-Gly-OH, in both methods of administration and three dosages) no ulcerous lesions were macroscopically observed. Gastric mucous membrane was uniformly epithelialized and had a pink color. Epithelization of the ulcerous lesion was observed. No ulcerous lesions were found in the mucous membrane, only zones with glandular cavities covered with proliferating epithelium were observed. In the underlying mucous stroma a fibrosis tissue with a regular pattern of collagen fibers and a mild inflammatory lymphoid and histiocytal infiltration was observed.

It should be noted that the most pronounced effect was observed in Groups 3b, 3c, 3e and 3f where the animals were treated with the means on the basis of the pharmaceutical composition comprising the peptide H-Glu-Asp-Gly-OH, administered subcutaneously and intragastrically, respectively, in the doses of 2,5 µg/kg and 10 µg/kg. However, subcutaneous or intramuscular administration of the means on the basis of the pharmaceutical composition comprising the peptide H-Glu-Asp-Gly-OH to the animals in the dose of 1,0 µg/kg (Groups 3a and 3d) also contributed to a quicker epithelialization of the ulcerous lesion but 2-3 days later as compared to the animals treated with the pharmaceutical composition comprising the peptide H-Glu-Asp-Gly-OH in higher doses. This prompts a conclusion on the efficiency of the means on the basis of the pharmaceutical composition comprising the peptide H-Glu-Asp-Gly-OH in the dose of 1,0 µg/kg for the prevention of *Helicobacter pylori-induced* gastric ulcer disease development.

Administration of Klacid (Group 4) caused the same changes.

Confocal microscopy and computer analysis of microscopic images showed as follows.

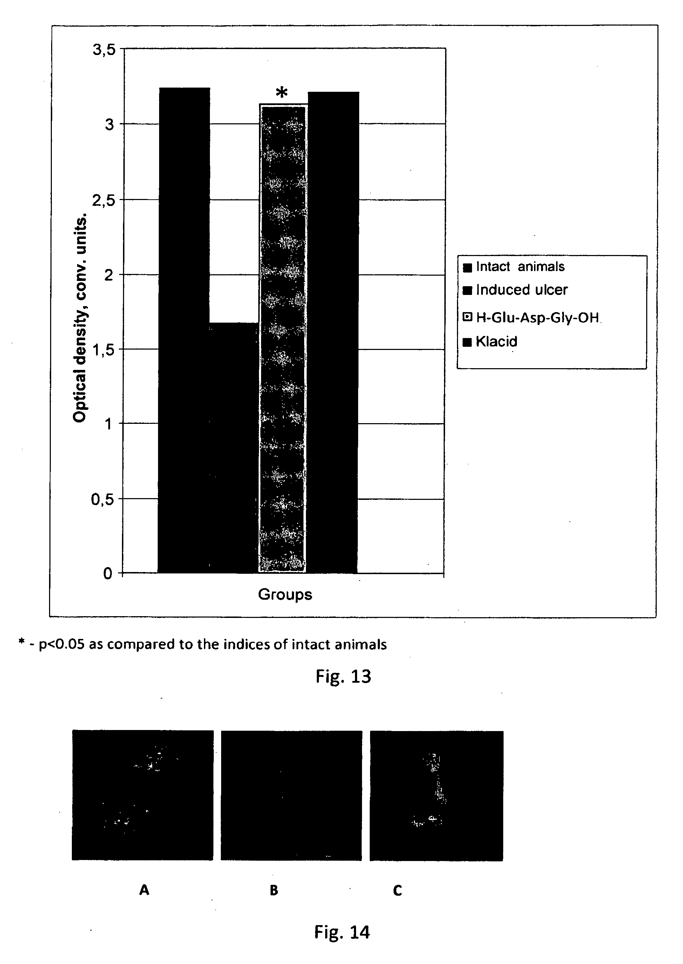

In Group 1 (intact animals) gastric epithelial cell mitochondria has a normal round or oval shape with a notable fluorescence of cristae and matrix. Mitochondrial GFP expression was significant and made in average, by optical density value, 3.12±0.22 conv. units by the 7^{th} day, 3.17±0.18 conv. units by the 15^{th} day and 3.19±0.18 conv. units by the 21^{st} day (Fig. 13-16).

Ulcer induction (Group 2) caused a pronounced disintegration of mitochondria by the 7^{th} day, accompanied by disruption of the mitochondrial membrane and cristae, wherein the fluorescence intensity of mitochondrial GFP significantly declined (Fig. 13), which caused a drastic decrease in marker expression indices (0.54±0.02 conv. units).

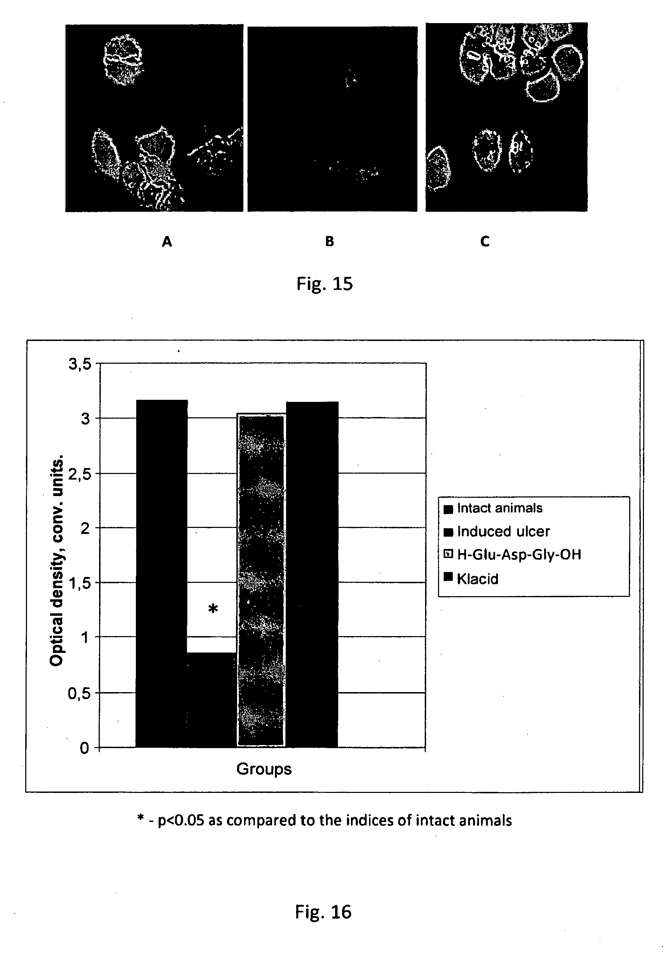

By the 15^{th} day partial restoration of mitochondrial structure was observed in mitochondria extracted from tissue samples by the index of optical density of mitochondrial GFP expression (0.87±0.04 conv. units, Fig. 14, 16).

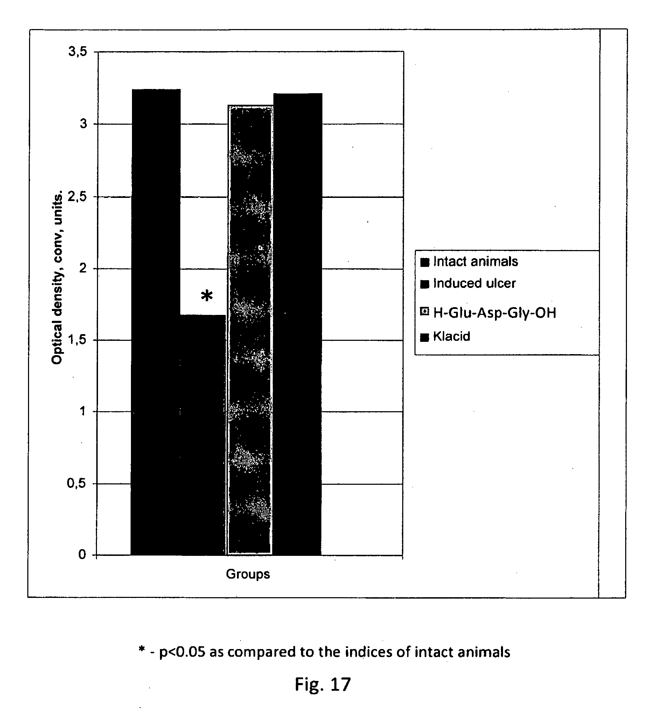

By the 21^{st} day an even more pronounced restoration of mitochondrial GFP expression indices was observed (optical density = 1.67±0.09 conv. units, Fig. 15, 17).

Under the effect of the means on the basis of the pharmaceutical composition comprising the peptide H-Glu-Asp-Gly-OH administered using both methods and in three studied doses, signs of outline and structure integrity were observed in the mitochondria in all periods of study. Dynamics of the mitochondrial GFP expression was as follows: 2.86±0.21 conv. units on the 7^{th} day, 3.03±0.16 conv. units on the 15^{th} day, 3.16±0.18 conv. units on the 21^{st} day (Fig. 13-17).

Klacid antibiotic also exerted similar effects (Fig. 13-17).

### Example 5. Study of toxicity of peptide H-Glu-Asp-Gly-OH

General toxicity of peptide H-Glu-Asp-Gly-OH was studied in accordance with the requirements of "Manual for Experimental (Pre-Clinical) Study of New Pharmacological Substances" (2000): acute toxicity in case of a single administration of the preparation, as well as subacute and chronic toxicity in case of a long-term administration of the peptide.

Acute toxicity study was carried out on 66 white mongrel male mice with body weight 20-22 g. The animals were randomly subdivided into 6 equal groups. The preparation was administered to the animals once, intramuscularly in the doses of 1, 2, 3, 4, 5 mg/kg in 0,25 ml of sterile 0,9% NaCl solution. Control animals were treated with 0,9% NaCl solution in the same volume.

Subacute toxicity study was carried out on 64 white mongrel male rats with body weight 180-220 g. Experimental animals received the preparation daily, once a day for 90 days in the doses of 1 µg/kg, 0,1 mg/kg, 1 mg/kg in 0,5 ml of sterile 0,9% NaCl solution. Control animals were treated with sterile 0,9% NaCl solution in the same volume. Before the administration of the preparation, as well as on the 30^{th}, 60^{th} and 90^{th} day after the beginning of the administration of the preparation peripheral blood morphology and properties were studied in the animals. Upon completion of the experiment biochemical and coagulology indices of the blood were evaluated.

Chronic toxicity was studied for 6 months basing on the duration of the recommended clinical administration of the preparation on 92 male guinea pigs with body weight 310-350 g. Experimental animals were treated with the peptide daily, once a day, intramuscularly for 6 months in the doses of 1 µg/kg, 0,1 mg/kg, 1 mg/kg in 0,5 ml of sterile 0,9% NaCl solution. Control animals received sterile 0,9% NaCl solution according to the same scheme and in the same volume. The following indices were identified in the peripheral blood of the animals using traditional methods: quantity of erythrocytes, hemoglobin, reticulocytes, thrombocytes, leukocytes, leukocyte formula, erythrocyte sedimentation rate (ESR), erythrocyte resistance. Further, total protein was identified in the blood serum by the Lowry method, as well as potassium and sodium using plasma spectrophotometry method. After completion of the experiment a pathomorphology study of the brain and bone marrow, spinal cord ganglia, thyroid gland, parathyroid glands, adrenal glands, testis, pineal gland, heart, lungs, aorta, liver, kidney, urinary bladder, pancreas, stomach, small intestine, large intestine, thymus, spleen, lymph nodes, bone marrow.

Study of acute toxicity has shown that a single administration of the studied peptide to animals in the dose that exceeds the therapeutic dose recommended for clinical use by more than 5000 times does not cause toxic reactions, which points out the wide therapeutic range of the preparation. Study of subacute and chronic toxicity of the peptide shows no side effects in case of a long-term administration of the preparation in the doses that exceed the therapeutic one by 100-1000 times. The study of the peptide effect on the morphological composition of guinea pig blood showed an increase in the amount of leukocytes in 3 months from the beginning of the administration of the preparation, the index was normalized by the 6^{th} month of observation (see Table). The other indices of the blood morphology in animals were largely unchanged. No significant effect of the preparation on the ESR, erythrocyte resistance and biochemical indices of the blood serum was revealed. Evaluation of the general status of the animals, of morphological and biochemical indices of the peripheral blood, morphology of the internal organs, cardiovascular and respiratory system status, liver and kidney function did not show any pathologic changes in the organism. The absence of general toxicity enables recommending the pharmaceutical composition comprising the peptide H-Glu-Asp-Gly-OH as its active base for clinical studies. Thus, as it may be seen from the materials that illustrate the invention, an advantage of the means on the basis of the pharmaceutical composition comprising the peptide H-Glu-Asp-Gly-OH is that contrary to known and traditional medicinal means, in particular Klacid medication (clarithromycin), it has a specific activity. Besides, administration of the means on the basis of the pharmaceutical composition comprising an amount, effective on *Helicobacter pylori,* of the peptide with formula H-Glu-Asp-Gly-OH as its active base in the dose of 1 µg to 10 µg per 1 kg of body weight orally, daily, 3 times a day for 10-20 days or parenterally, daily, once a day for 10 days exerts a protective effect on mitochondrial mechanisms of *Helicobacter pylori*-induced apoptosis, which makes it possible to suggest using this means for the treatment of gastroduodenal diseases caused by *Helicobacter pylori.*

| Index | Administration of peptide H-Glu-Asp-Gly-OH (1 µg/kg) | | | |
|---|---|---|---|---|
| | 3 months | | 6 months | |
| | Control (n=24) | Peptide (n=24) | Control (n=24) | Peptide (n=24) |
| Erythrocytes, x10¹²/l | 5,3±0,6 | 5,2±0,1 | 5,4±0,3 | 5,2±0,7 |
| Hemoglobin, g/l | 14,2±1,4 | 14,4±0,9 | 14,5±1,3 | 14,8±1,1 |
| Reticulocytes, % | 1,3±0,07 | 1,1±0,08 | 1,1±0,05 | 1,2±0,04 |
| Thrombocytes, x10⁹/l | 143,7±7,9 | 143,1±4,5 | 144,5±8,6 | 142,8±6,2 |
| Leukocytes, x10⁹/l | 9,4±0,5 | 11,8±0,2* | 9,6±0,5 | 10,3±0,6 |
| Stab neutrophils, % | 0,31±0,04 | 0,30±0,02 | 0,33±0,04 | 0,35±0,01 |
| Segmented neutrophils, % | 45,8±2,1 | 43,9±1,8 | 46,2±3,5 | 45,4±3,0 |
| Eosinophils % | 0,69±0,05 | 0,67±0,06 | 0,72±0,04 | 0,73±0,04 |
| Basophils % | 0,61±0,04 | 0,63±0,03 | 0,72±0,03 | 0,65±0,08 |
| Monocytes, % | 2,5±0,02 | 2,6±0,01 | 2,6±0,06 | 2,4±0,04 |
| Lymphocytes, % | 48,9±2,5 | 51,3±2,2 | 51,3±2,7 | 49,7±1,9 |
| ESR, mm/hour | 1,69±0,05 | 1,98±0,09 | 2,01±0,05 | 1,85±0,06 |
| Erythrocyte resistance, % NaCl | | | | |
| - maximum | 0,41±0,02 | 0,42±0,02 | 0,42±0,04 | 0,43±0,02 |
| - minimum | 0,32±0,05 | 0,31±0,01 | 0,34±0,04 | 0,33±0,06 |
| Total protein in blood serum, g/l | 72,9±3,1 | 73,6±2,8 | 73,1±3,4 | 72,3±2,9 |
| Sodium in blood serum, mmole/l | 153,9±5,7 | 155,6±4,7 | 155,5±6,2 | 155,2±4,9 |
| Potassium in blood serum, mmole/l | 5,1±2,3 | 5,3±2,0 | 5,2±2,1 | 5,1±2,5 |

| | | | | |
|---|---|---|---|---|
| *- P<0,05 as compared to the control | | | | |

## Claims

1. A pharmaceutical composition comprising an amount, effective on *Helicobacter pylori,* of peptide with formula H-Glu-Asp-Gly-OH as the active substance, and a pharmaceutically acceptable carrier, for use in the treatment of *Helicobacter pylori-*induced gastroduodenal diseases, by inhibiting mitochondrial mechanisms of *Helicobacter pylori-induced* apoptosis in gastric epitheliocytes.

2. The pharmaceutical composition for use of claim 1, **characterized in that** it comprises the active substance in the amount of 1 µg/kg to 10 µg/kg.

3. The pharmaceutical composition for use of claim 1 or 2, **characterized in that** it exists in the form suitable for oral or parenteral administration.

4. A peptide with formula H-Glu-Asp-Gly-OH as the active substance for use in the treatment of *Helicobacter pylori*-induced gastroduodenal diseases.

5. The peptide for use of claim 4, as the active substance in the treatment of *Helicobacter pylori-induced* gastroduodenal diseases **characterized in that** is administered in the amount of 1 µg/kg to 10 µg/kg.

6. The peptide for use of claim 5, **characterized in that** the administration is orally or parenterally.

7. The composition for use of claim 1 **characterized by** administering an amount, effective on *Helicobacter pylori,* of the peptide with formula H-Glu-Asp-Gly-OH in the dose of 1 µg to 10 µg per 1 kg of body weight orally, daily, 3 times a day for 10-20 days, or parenterally, daily, once a day for 10 days.

8. The composition for use of claim 7, **characterized in that** the administration is orally or parenterally.

## Patentansprüche

1. Eine pharmazeutische Zusammensetzung umfassend eine auf Helicobacter pylori wirksame Menge an Peptid mit Formel H-Glu-Asp-Gly-OH als die aktive Substanz und einen pharmazeutisch annehmbaren Träger, zur Verwendung in der Behandlung von durch Helicobacter pylori induzierten gastroduodenalen Krankheiten, durch Inhibierung mitochondrialer Mechanismen von durch Helicobacter pylori induzierter Apoptose in Epithelzellen des Magens.

2. Die pharmazeutische Zusammensetzung zur Verwendung von Anspruch 1, **dadurch gekennzeichnet, dass** sie die aktive Substanz in der Menge von 1 µg / kg bis 10 µg / kg umfasst.

3. Die pharmazeutische Zusammensetzung zur Verwendung von Anspruch 1, **dadurch gekennzeichnet, dass** sie in der für orale oder parenterale Verabreichung geeigneten Form vorliegt.

4. Ein Peptid mit Formel H-Glu-Asp-Gly-OH als die aktive Substanz, zur Verwendung in der Behandlung von durch Helicobacter pylori induzierten gastroduodenalen Krankheiten.

5. Das Peptid zur Verwendung von Anspruch 4 als die aktive Substanz in der Behandlung von durch Helicobacter pylori induzierten gastroduodenalen Krankheiten, **dadurch gekennzeichnet, dass** es in der Menge von 1 µg / kg bis 10 µg / kg verabreicht wird.

6. Das Peptid zur Verwendung von Anspruch 4, **dadurch gekennzeichnet, dass** die Verabreichung oral oder parenteral erfolgt.

7. Die pharmazeutische Zusammensetzung zur Verwendung von Anspruch 1, **dadurch gekennzeichnet, dass** eine auf Helicobacter pylori wirksame Menge des Peptides mit Formel H-Glu-Asp-Gly-OH in der Dosis von 1 µg bis 10 µg pro 1 kg Körpergewicht oral 3mal täglich während 10-20 Tagen, oder parenteral täglich einmal pro Tag während 10 Tagen verabreicht wird.

8. Die Zusammensetzung zur Verwendung von Anspruch 7, **dadurch gekennzeichnet, dass** die Verabreichung oral oder parenteral erfolgt.

## Revendications

1. Une composition pharmaceutique comprenant une quantité efficace sur Helicobacter pylori de peptide de formule H-Glu-Asp-Gly-OH comme la substance active et un support pharmaceutiquement acceptable, pour l'utilisation dans le traitement de maladies gastroduodénales induites par l'Helicobacter pylori, par inhibition de mécanismes mitochondriaux de l'apoptose induite par Helicobacter pylori dans des cellules épithéliales gastriques.

2. La composition pharmaceutique pour l'utilisation de la revendication 1, **caractérisée en ce qu'**elle comprend la substance active en une quantité de 1µg/kg à 10 µg/kg.

3. La composition pharmaceutique pour l'utilisation de la revendication 1 ou 2, **caractérisée en ce qu'**elle existe sous une forme appropriée pour une administration par voie orale ou par voie parentérale.

4. Un peptide ayant la formule H-Glu-Asp-Gly-OH comme la substance active pour l'utilisation dans le traitement de maladies gastroduodénales induites par l'Helicobacter pylori.

5. Le peptide pour l'utilisation de la revendication 4 comme la substance active dans le traitement de maladies gastroduodénales induites par l'Helicobacter pylori, **caractérisé en ce qu'**il soit administré en une quantité de 1 µg/kg à 10 µg/kg.

6. Le peptide pour l'utilisation de la revendication 5, **caractérisé en ce que** l'administration a lieu par voie orale ou par voie parentérale.

7. La composition pour l'utilisation de la revendication 1, **caractérisée en ce qu'**une quantité efficace sur Helicobacter pylori du peptide de formule H-Glu-Asp-Gly-OH est administrée en dose de 1 µg à 10 µg par 1 kg de poids du corps, par voie orale, quotidiennement, 3 fois par jour pendant 10-20 jours, ou par voie parentérale, quotidiennement, une fois par jour pendant 10 jours.

8. La composition pour l'utilisation de la revendication 7, **caractérisé en ce que** l'administration a lieu par voie orale ou par voie parentérale
